# EUROPEAN PATENT APPLICATION

(11) **EP 4 249 045 A1**
(43) Date of publication of application: **27.09.2023**
(21) Application number: 22164409.9
(22) Date of filing: 25.03.2022
(51) Int. Cl.: A61N 1/372, G16H 20/10, G16H 20/40, G16H 40/63, G16H 40/67, G16H 80/00

(54) **IMPLANT COMMUNICATION SYSTEM AND METHOD FOR COMMUNICATING WITH AN IMPLANTABLE MEDICAL DEVICE**

(71) Applicant: BIOTRONIK SE & Co. KG, 12359 Berlin (DE)
(72) Inventor: Stephan, Enrico, 13055 Berlin (DE); Tietz, Marko, 10243 Berlin (DE)
(74) Representative: Biotronik Corporate Services SE

(57) **Abstract**

The invention relates to an implant communication system (1), wherein the consumer mobile communication device (14) is configured to submit a data read-out request (R) to the implant communication device (10), wherein the implant communication device (10) is configured to read-out the data (D1) specified in the data read-out request (R) from the implantable medical device (12) and to send the read-out data (D1) to the consumer mobile communication device (14), and wherein the consumer mobile communication device (14) is configured to display the read-out data (D1) received from the implant communication device (10) on the display device (14a). Furthermore, the invention relates to a computer implemented method for communicating with an implantable medical device, a computer program and a computer readable data carrier.

## Description

The invention relates to an implant communication system. Furthermore, the invention relates to a computer implemented method for communicating with an implantable medical device.

US 2020/0357513 A1 discloses techniques for remote monitoring of a patient and corresponding medical device(s). The remote monitoring comprises providing an interactive session configured to allow a user to navigate a plurality of sub sessions, determining a first set of data items in accordance with a first sub session, the first set of data items including the image data, determining a second set of data items in accordance with a second sub session of the interactive session, determining, based at least in part on the first set of data items and the second set of data items, an abnormality, and outputting a post-implant report of the interactive session.

WO 2007/070669 A2 discloses a patient management device for portably interfacing with a plurality of implantable medical devices and method thereof is presented. Permission to interrogate one or more implantable medical devices is authenticated. Patient device data is individually exchanged through interrogation of at least one authenticated implantable medical device through short range telemetry. External device data is exchanged via communication with at least one external device through long range telemetry. At least one of the patient device and external device data is maintained contemporaneously to execution of operations to perform one or more of relay, processing, and outputting of the patient device and external device data subsequent to the interrogation of the implantable medical device.

Conventional implant systems hence comprise a programmer capable of reading out data from and writing data to an implantable medical device. Such proprietary programming devices can also be used solely for reading out data from the implantable medical device.

If a patient wearing an implantable medical device is admitted to a hospital and/or medical practice due to an acute medical condition data is read out from the implantable medical device by means of said programmer by a trained expert and/or medical practitioner in order to evaluate medical parameters of the patient recorded by the implantable medical device as well as technical parameters of the implantable medical device in order to evaluate if the implantable medical device is operating normally or whether a technical issue is present.

Programmers however are not universally available at all medical facilities such as hospitals and/or medical practices. Furthermore, such programmers may be difficult to use for untrained medical staff, acceptance of such devices is generally limited.

It is therefore an object of the present invention to provide an improved implant communication system that is usable not only by medical practitioners but also by untrained medical staff and is generally widely accepted and universally available.

The object is solved by an implant communication system having the features of claim 1.

Furthermore, the object is solved by a computer implemented method for communicating with an implantable medical device having the features of claim 10.

In addition, the object is solved by a computer program with program code to perform the method according to the present invention when the computer program is executed on a computer having the features of claim 13.

Moreover, the object is solved by a computer readable data carrier with program code of a computer program to perform the method according to the present invention when the computer program is executed on a computer having the features of claim 14.

Further developments and advantageous embodiments are defined in the dependent claims.

The present invention provides an implant communication system, comprising an implant communication device configured to read-out data from an implantable medical device. In addition, the implant communication system comprises a consumer mobile communication device, in particular a smartphone or tablet computing device, comprising a display device, wherein the consumer mobile communication device is configured to communicate with the implant communication device in a frequency band and/or using a communication protocol supported by the consumer mobile communication device.

The consumer mobile communication device is configured to submit a data read-out request to the implant communication device, wherein the implant communication device is configured to read-out the data specified in the data read-out request from the implantable medical device and to send the read-out data to the consumer mobile communication device, and wherein the consumer mobile communication device is configured to display the read-out data received from the implant communication device on the display device.

Furthermore, the present invention provides a computer implemented method for communicating with an implantable medical device. The method comprises providing an implant communication device configured to read-out data from an implantable medical device. Moreover, the method comprises providing a consumer mobile communication device, in particular a smartphone or tablet computing device, comprising a display device, wherein the consumer mobile communication device communicates with the implant communication device in a frequency band and/or using a communication protocol supported by the consumer mobile communication device.

The method additionally comprises submitting a data read-out request to the implant communication device by means of the consumer mobile communication device and reading-out the data specified in the data read-out request from the implantable medical device by means of the implant communication device.

Furthermore, the method comprises sending the read-out data to the consumer mobile communication device by means of the implant communication device, and displaying the read-out data received from the implant communication device on the display device by means of the consumer mobile communication device.

An idea of the present invention is to provide medical staff with an easy to use and widely available tool for accessing a patient's implantable medical device for the purposes of reading out data related to a condition of the patient, i.e. medical and/or technical parameters recorded by the implantable medical device.

The conventionally used proprietary device for accessing the implantable medical device is thus replaced by a consumer mobile communication device such as a smart phone or tablet computing device in combination with the implant communication device connectable to the consumer mobile communication device.

An app installed in the consumer mobile communication device allows connecting to the implant communication device and to read-out data from the implantable medical device and in a fashion that can be performed by a non-expert, i.e. not specifically trained medical staff. The implant communication device comprises a logic unit as well as firmware allowing it access and read-out data from the implantable medical device.

The implantable medical device may be formed by a purely therapeutic implant, an implant with therapeutic and diagnostic functions and/or an implant with therapeutic and diagnostic functions.

An example of a purely therapeutic implant / implantable medical device is e.g. a stimulator / electrode for deep brain stimulation. The therapy consists of the delivery of pulse trains without collecting diagnostic data from the stimulator.

An example of a purely diagnostic implant is e.g. a cardiac rhythm monitor. The diagnostic function consists of continuous recording of the patient's ECG and automatic evaluation of abnormalities of the heart rhythm. If such are detected, an ECG recording is stored and typically automatically transmitted to a remote monitoring system.

An example of an implant with therapeutic and diagnostic functions is e.g. a cardiac pacemaker. The pacemaker is typically implanted subcutaneously in the upper right thoracic region and the electrode is placed in the patient's heart via a large vein. The therapeutic function consists of delivering stimulation pulses to trigger a cardiac action, provided there is no spontaneous cardiac action in the patient. The diagnostic function consists, for example, in the continuous recording of the patient's ECG and automatic evaluation of abnormalities of the heart rhythm. If such are detected, an ECG recording is stored and typically automatically transmitted to a remote monitoring system.

According to an aspect of the invention, the implant communication device comprises a communication adapter for transferring data between the implantable medical device, in particular a pacemaker, a defibrillator and/or a neuro-stimulator, and the consumer mobile communication device and a logic unit for interrogating the implantable medical device.

Providing the communication adapter and the logic unit as part of the implant communication device advantageously enables usage of the consumer mobile communication device as a user interface for interacting with the implantable mobile device since the consumer mobile convocation device does not need to contain said components.

According to a further aspect of the invention, the communication adapter comprises a MICS telemetry interface, a Bluetooth LE interface and/or a near field telemetry interface, in particular an inductive coil, for data transfer between the communication adapter and the implantable medical device, and a consumer mobile communication device wireless transmission interface, in particular a Bluetooth, Bluetooth LE and/or WLAN interface, for data transfer between the communication adapter and the consumer mobile communication device.

The communication adapter converts a communication standard of a mobile device, e.g. Bluetooth or Wi-Fi, to a communication of the implantable medical device, e.g. a MICS band telemetry. Using MICS band telemetry, the implantable medical device can thus communicate easily and inexpensively with a mobile device such as a smart phone.

According to a further aspect of the invention, the communication adapter is configured to be powered by an energy source, in particular a battery or an inductive energy source, wherein the communication adapter is configured to transfer data sent via the MICS telemetry interface, the Bluetooth LE interface and/or the near field telemetry interface, by the implantable medical device to the consumer mobile communication device via the consumer mobile communication device, and to transfer data sent via the consumer mobile communication device wireless transmission interface by the consumer mobile communication device to the implantable medical device via the MICS telemetry interface, the Bluetooth LE interface and/or the near field telemetry interface.

The communication adapter of the implant communication device thus advantageously serves as an intermediary device interposed between the consumer mobile communication device and the implantable medical device in order to facilitate communication between the consumer mobile communication device and the implantable medical device.

According to a further aspect of the invention, the implant communication device is adapted such that the energy source is replaceable or rechargeable. This enables ease of use and facilitates replacement of the energy source when necessary.

According to a further aspect of the invention, the communication adapter comprises or is connected to at least a MICS-band antenna, a Bluetooth-band antenna, a WLAN-band antenna and/or a near field antenna, in particular an inductive coil. This provides the advantage of antenna diversity.

According to a further aspect of the invention, the implant communication device comprises a non-volatile buffer configured to at least temporarily store information and transfer it to the implantable medical device and/or the consumer mobile communication device at a later time. The communication adapter thus does not have to be connected to the implant and the mobile device at the same time for its function, i.e. it contains a non-volatile buffer to temporarily store information and forward it at a later time.

According to a further aspect of the invention, the implant communication system comprises a remote server, the consumer mobile communication device is configured to transfer the read-out data received from the implant communication device to the remote server via a network connection, and wherein the consumer mobile communication device comprises an encryption unit, in particular a hardware- and/or software-based encryption unit, configured to encrypt a communication between the consumer mobile communication device and the remote server. The communication between the mobile device and the implantable medical device can thus be rendered more secure.

According to a further aspect of the invention, the implant communication device comprises no display device, and wherein the implant communication device has read-only access to the implantable medical device. Due to the fact that the implant communication device comprises no display device it can be built having small dimensions. It is thus an easily portable add-on-device for the consumer mobile communication device.

According to a further aspect of the invention, the data transferred to the remote server is analyzed to evaluate medical parameters of a patient recorded by the implantable medical device and/or technical parameters of the implantable medical device, in particular a current operating mode, a state of charge of a battery and/or an event log of the implantable medical device. Said analysis of the data transferred to the remote server is preferably performed by trained medical staff /experts. It is thus possible to discriminate if a condition of the patient is related to either medical parameters of the patient or technical parameters of the implantable medical device. The evaluated data, i.e. the result of said data evaluation is sent back from the remote server to the consumer mobile communication device.

The herein described features of the implant communication system are also disclosed for the computer implemented method for communicating with an implantable medical device and vice versa.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Fig. 1: shows a schematic view of an implant communication system according to a preferred embodiment of the invention; and
- Fig. 2: shows a flowchart of a computer implemented method for communicating with an implantable medical device according to the preferred embodiment of the invention.

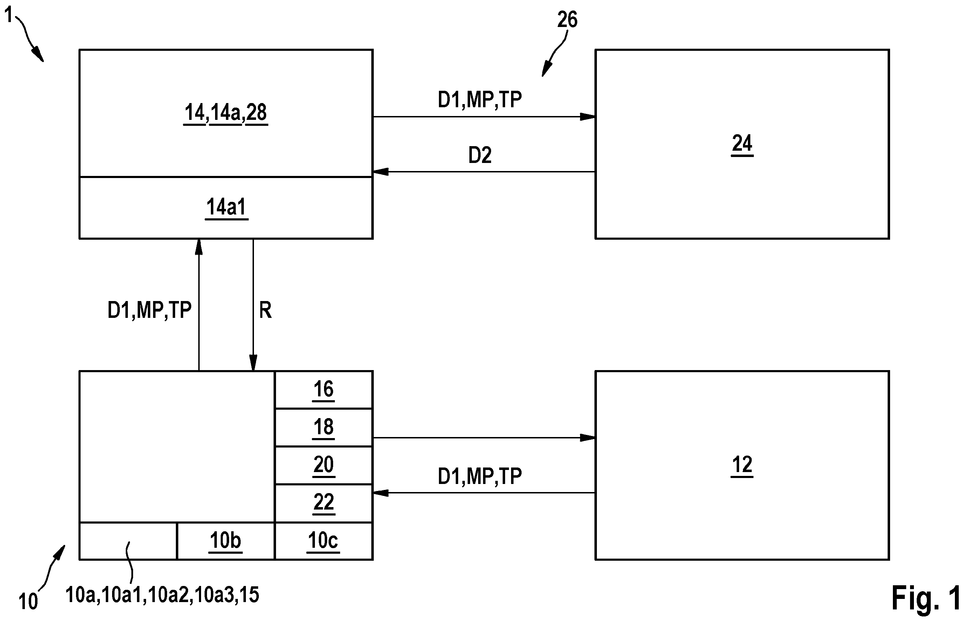

The implant communication system 1 of Fig. 1 comprises an implant communication device 10 configured to read-out data D1 from an implantable medical device 12.

Furthermore, the implant communication system 1 comprises a consumer mobile communication device 14, in particular a smartphone or tablet computing device, comprising a display device 14a, wherein the consumer mobile communication device 14 is configured to communicate with the implant communication device 10 in a frequency band and/or using a communication protocol supported by the consumer mobile communication device 14.

In addition, the consumer mobile communication device 14 is configured to submit a data read-out request R to the implant communication device 10, wherein the implant communication device 10 is configured to read-out the data D1 specified in the data read-out request R from the implantable medical device 12 and to send the read-out data D1 to the consumer mobile communication device 14. The consumer mobile communication device 14 is further configured to display the read-out data D1 received from the implant communication device 10 on the display device 14a.

The implant communication device 10 comprises a communication adapter 10a for transferring data D1 between the implantable medical device 12, in particular a pacemaker, a defibrillator and/or a neuro-stimulator, and the consumer mobile communication device 14 and a logic unit 10b for interrogating the implantable medical device 12.

The communication adapter 10a comprises a MICS telemetry interface 10a1, a Bluetooth LE interface 10a2 and/or a near field telemetry interface 10a3, in particular an inductive coil, for data D1 transfer between the communication adapter 10a and the implantable medical device 12, and a consumer mobile communication device wireless transmission interface 14a1, in particular a Bluetooth, Bluetooth LE and/or WLAN interface, for data D1 transfer between the communication adapter 10a and the consumer mobile communication device 14.

The communication adapter 10a is configured to be powered by an energy source 15, in particular a battery or an inductive energy source 15, wherein the communication adapter 10a is configured to transfer data D1 sent via the MICS telemetry interface 10a1, the Bluetooth LE interface 10a2 and/or the near field telemetry interface 10a3, by the implantable medical device 12 to the consumer mobile communication device 14 via the consumer mobile communication device 14, and to transfer data D1 sent via the consumer mobile communication device wireless transmission interface 14a1 by the consumer mobile communication device 14 to the implantable medical device 12 via the MICS telemetry interface 10a1, the Bluetooth LE interface 10a2 and/or the near field telemetry interface 10a3.

The implant communication device 10 is adapted such that the energy source 15 is replaceable or rechargeable. Furthermore, the communication adapter 10a comprises or is connected to at least a MICS-band antenna 16, a Bluetooth-band antenna 18, a WLAN-band antenna 20 and/or a near field antenna 22, in particular an inductive coil.

The implant communication device 10 comprises a non-volatile buffer 10c configured to at least temporarily store information and transfer it to the implantable medical device 12 and/or the consumer mobile communication device 14 at a later time.

The implant communication system 1 further comprises a remote server 24, wherein the consumer mobile communication device 14 is configured to transfer the read-out data D1 received from the implant communication device 10 to the remote server 24 via a network connection 26, and wherein the consumer mobile communication device 14 comprises an encryption unit 28, in particular a hardware- and/or software-based encryption unit 28, configured to encrypt a communication between the consumer mobile communication device 14 and the remote server 24.

Said analysis of the data D1 transferred to the remote server 24 is preferably performed by trained medical staff /experts. It is thus possible to discriminate if a condition of the patient is related to either medical parameters MP of the patient or technical parameters TP of the implantable medical device 12. Evaluated data D2, i.e. the result of said data evaluation is sent back from the remote server 24 to the consumer mobile communication device 14.

The implant communication device 10 comprises no display device. Moreover, the implant communication device 10 has read-only access to the implantable medical device 12.

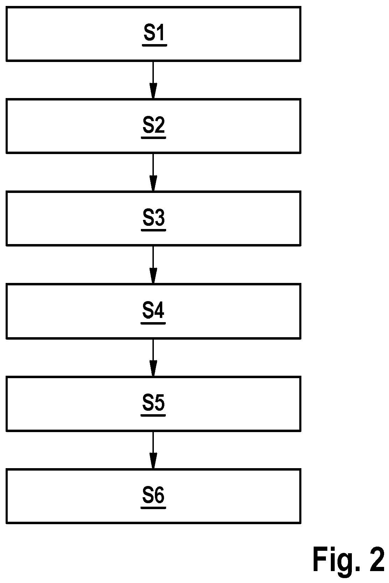

Fig. 2 shows a flowchart of a computer implemented method for communicating with an implantable medical device according to the preferred embodiment of the invention.

The computer implemented method for communicating with an implantable medical device 12 comprises providing S1 an implant communication device 10 configured to read-out data D1 from an implantable medical device 12 and providing S2 a consumer mobile communication device 14, in particular a smartphone or tablet computing device, comprising a display device 14a, wherein the consumer mobile communication device 14 communicates with the implant communication device 10 in a frequency band and/or using a communication protocol supported by the consumer mobile communication device 14.

The method further comprises submitting S3 a data read-out request R to the implant communication device 10 by means of the consumer mobile communication device 14 and reading-out S4 the data D1 specified in the data read-out request R from the implantable medical device 12 by means of the implant communication device 10.

In addition, the method comprises sending S5 the read-out data D1 to the consumer mobile communication device 14 by means of the implant communication device 10, and displaying S6 the read-out data D1 received from the implant communication device 10 on the display device 14a by means of the consumer mobile communication device 14.

The consumer mobile communication device 14 transfers the read-out data D1 received from the implant communication device 10 to a remote server 24 via a network connection 26.

Moreover, the data D1 to be transferred is encrypted by means of an encryption unit 28, in particular a hardware- and/or software-based encryption unit 28, encrypting a communication between the consumer mobile communication device 14 and the remote server 24.

The data D1 transferred to the remote server 24 is analyzed to evaluate medical parameters MP of a patient recorded by the implantable medical device 12 and/or technical parameters TP of the implantable medical device 12, in particular a current operating mode, a state of charge of a battery and/or an event log of the implantable medical device 12.

### Reference Signs

- 1: implant communication system
- 10: implant communication device
- 10a: communication adapter
- 10a1: MICS telemetry interface
- 10a2: Bluetooth LE interface
- 10a3: near field telemetry interface
- 10b: logic unit
- 10c: non-volatile buffer
- 12: implantable medical device
- 14: consumer mobile communication device
- 14a: display device
- 14a1: consumer mobile communication device wireless transmission interface
- 15: energy source
- 16: MICS-band antenna
- 18: Bluetooth-band antenna
- 20: WLAN-band antenna
- 22: near field antenna
- 24: remote server
- 26: network connection
- 28: encryption unit
- D1: data
- D2: evaluated data
- MP: medical parameters
- TP: technical parameters
- R: read-out request
- S1-S6: method steps

## Claims

1. Implant communication system (1), comprising:
an implant communication device (10) configured to read-out data (D1) from an implantable medical device (12); and
a consumer mobile communication device (14), in particular a smartphone or tablet computing device, comprising a display device (14a), wherein the consumer mobile communication device (14) is configured to communicate with the implant communication device (10) in a frequency band and/or using a communication protocol supported by the consumer mobile communication device (14), wherein the consumer mobile communication device (14) is configured to submit a data read-out request (R) to the implant communication device (10), wherein the implant communication device (10) is configured to read-out the data (D1) specified in the data read-out request (R) from the implantable medical device (12) and to send the read-out data (D1) to the consumer mobile communication device (14), and wherein the consumer mobile communication device (14) is configured to display the read-out data (D1) received from the implant communication device (10) on the display device (14a).

2. Implant communication system of claim 1, wherein the implant communication device (10) comprises a communication adapter (10a) for transferring data (D1) between the implantable medical device (12), in particular a pacemaker, a defibrillator and/or a neuro-stimulator, and the consumer mobile communication device (14) and a logic unit (10b) for interrogating the implantable medical device (12).

3. Implant communication system of claim 2, wherein the communication adapter (10a) comprises a MICS telemetry interface (10a1), a Bluetooth LE interface (10a2) and/or a near field telemetry interface (10a3), in particular an inductive coil, for data (D1) transfer between the communication adapter (10a) and the implantable medical device (12), and
a consumer mobile communication device wireless transmission interface (14a1), in particular a Bluetooth, Bluetooth LE and/or WLAN interface, for data (D1) transfer between the communication adapter (10a) and the consumer mobile communication device (14).

4. Implant communication system of claim 2, wherein the communication adapter (10a) is configured to be powered by an energy source (15), in particular a battery or an inductive energy source (15), wherein the communication adapter (10a) is configured to transfer data (D1) sent via the MICS telemetry interface (10a1), the Bluetooth LE interface (10a2) and/or the near field telemetry interface (10a3), by the implantable medical device (12) to the consumer mobile communication device (14) via the consumer mobile communication device (14), and to transfer data (D1) sent via the consumer mobile communication device wireless transmission interface (14a1) by the consumer mobile communication device (14) to the implantable medical device (12) via the MICS telemetry interface (10a1), the Bluetooth LE interface (10a2) and/or the near field telemetry interface (10a3).

5. Implant communication system of any one of claims 2 to 4, wherein the communication adapter (10a) comprises or is connected to at least a MICS-band antenna (16), a Bluetooth-band antenna (18), a WLAN-band antenna (20) and/or a near field antenna (22), in particular an inductive coil.

6. Implant communication system of any one of the preceding claims, wherein the implant communication device (10) is adapted such that the energy source (15) is replaceable or rechargeable.

7. Implant communication system of any one of the preceding claims, wherein the implant communication device (10) comprises a non-volatile buffer (10c) configured to at least temporarily store information and transfer it to the implantable medical device (12) and/or the consumer mobile communication device (14) at a later time.

8. Implant communication system of any one of the preceding claims, further comprising a remote server (24), wherein the consumer mobile communication device (14) is configured to transfer the read-out data (D1) received from the implant communication device (10) to the remote server (24) via a network connection (26), and wherein the consumer mobile communication device (14) comprises an encryption unit (28), in particular a hardware- and/or software-based encryption unit (28), configured to encrypt a communication between the consumer mobile communication device (14) and the remote server (24).

9. Implant communication system of any one of the preceding claims, wherein the implant communication device (10) comprises no display device, and wherein the implant communication device (10) has read-only access to the implantable medical device (12).

10. Computer implemented method for communicating with an implantable medical device (12) comprising the steps of:
providing (S1) an implant communication device (10) configured to read-out data (D1) from an implantable medical device (12); and
providing (S2) a consumer mobile communication device (14), in particular a smartphone or tablet computing device, comprising a display device (14a), wherein the consumer mobile communication device (14) communicates with the implant communication device (10) in a frequency band and/or using a communication protocol supported by the consumer mobile communication device (14);
submitting (S3) a data read-out request (R) to the implant communication device (10) by means of the consumer mobile communication device (14);
reading-out (S4) the data (D1) specified in the data read-out request (R) from the implantable medical device (12) by means of the implant communication device (10);
sending (S5) the read-out data (D1) to the consumer mobile communication device (14) by means of the implant communication device (10); and
displaying (S6) the read-out data (D1) received from the implant communication device (10) on the display device (14a) by means of the consumer mobile communication device (14).

11. Computer implemented method of claim 10, wherein the consumer mobile communication device (14) transfers the read-out data (D1) received from the implant communication device (10) to a remote server (24) via a network connection (26), and wherein the data (D1) to be transferred is encrypted by means of an encryption unit (28), in particular a hardware- and/or software-based encryption unit (28), encrypting a communication between the consumer mobile communication device (14) and the remote server (24).

12. Computer implemented method of claim 10 or 11, wherein the data (D1) transferred to the remote server (24) is analyzed to evaluate medical parameters (MP) of a patient recorded by the implantable medical device (12) and/or technical parameters (TP) of the implantable medical device (12), in particular a current operating mode, a state of charge of a battery and/or an event log of the implantable medical device (12).

13. Computer program with program code to perform the method of any one of claims 10 to 12 when the computer program is executed on a computer.

14. Computer readable data carrier with program code of a computer program to perform the method of any one of claims 10 to 12 when the computer program is executed on a computer.
